(19) Europäisches Patentamt
European Patent Office
Office européen des brevets

(11) Veröffentlichungsnummer: **0 207 461 B1**

(12) **EUROPÄISCHE PATENTSCHRIFT**

(45) Veröffentlichungstag der Patentschrift: **06.03.91**

(51) Int. Cl.[5]: **C07C 265/04**, C07C 263/00, C07C 271/16

(21) Anmeldenummer: **86108777.3**

(22) Anmeldetag: **27.06.86**

Teilanmeldung 90105691.1 eingereicht am 27/06/86.

# (54) Verfahren zur Herstellung von omega-Isocyanatoalkyl(meth)acrylaten.

(30) Priorität: **03.07.85 DE 3523692**

(43) Veröffentlichungstag der Anmeldung:
**07.01.87 Patentblatt 87/02**

(45) Bekanntmachung des Hinweises auf die Patenterteilung:
**06.03.91 Patentblatt 91/10**

(84) Benannte Vertragsstaaten:
**DE FR GB**

(56) Entgegenhaltungen:
**EP-A- 0 027 940**
**US-A- 2 718 516**

(73) Patentinhaber: **BASF Aktiengesellschaft**
**Carl-Bosch-Strasse 38**
**W-6700 Ludwigshafen(DE)**

(72) Erfinder: **Merger, Franz, Dr.**
**Max-Slevogt-Strasse 25**
**W-6710 Frankenthal(DE)**
Erfinder: **Schwarz, Wolfgang, Dr.**
**Wesostrasse 132**
**W-7507 Pfinztal(DE)**

**EP 0 207 461 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von ω-Isocyanatoalkyl-(meth)-acrylaten.

ω-Isocyanato-alkyl-(meth)acrylate der allgemeinen Formel 1

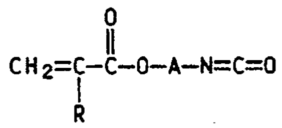 I,

in der A für einen Alkylenrest mit 2 bis 14 C-Atomen und R für H- oder $CH_3$-stehen, sind bekannt. Zur Herstellung von 2-Isocyanatoethyl-meth-acrylat (IEM) wird z.B. nach dem Verfahren der US-PS 2 718 516 Ethanolamin mit Chlorameisensäureethylester umgesetzt und das erhaltene Ethylhydroxyethylcarbamat mit Methacrylylchlorid acyliert und das dabei erhaltene Urethan dann in Gegenwart basischer Katalysatoren unter Erhitzen gespalten wird. IEM kann nach dem Verfahren der US-PS 2 821 544 auch durch Umsetzung von Methacrylylchlorid mit Ethanolaminhydrochlorid und Umsetzen des erhaltenen 2-Aminoethylmethacrylats mit Phosgen erhalten werden. Schließlich kommt man nach dem Verfahren der US-PS 4 278 809 auch durch Umsetzung von 2-Isopropenyloxazolin mit Phosgen zu IEM.

Da bei den bekannten Verfahren stark toxische Stoffe wie Phosgen und Chlorameisensäureester eingesetzt werden, ist ein großer sicherheitstechnischer Aufwand erforderlich. Außerdem entstehen bei diesen Verfahren erhebliche Mengen an Salzen, wie NaCl.

Es wurde nun gefunden, daß man ω-Isocyanatoalkyl-(meth)acrylate der allgemeinen Formel 1

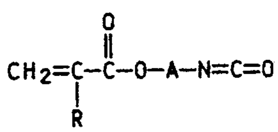 I,

in der A für einen Alkylenrest, einen Oxa-alkylenrest oder einen Polyoxaalkylenrest mit jeweils 2 bis 12 C-Atomen und R für H- oder $CH_3$-stehen, vorteilhaft herstellen kann, indem man

a) ω-Aminoalkanole, ω-Amino-oxa-alkanole oder ω-Amino-polyoxa-alkanole. die jeweils 2 bis 12 C-Atome enthalten, mit Harnstoff und einem Alkanol zu N-ω-Hydroxyalkyl-. N-ω-Hydroxy-oxa-alkyl- oder N-ω-Hydroxypolyoxa-alkyl-carbaminsäureestern umsetzt,

b) die N-ω-Hydroxy-carbaminsäureester durch Umsetzung mit (Meth)acrylsäurealkylestern oder (Meth)-acrylsäureanhydrid verestert und

c) die erhaltenen ω-Alkoxicarbamoyl-alkyl-, -oxa-alkyl- und -polyoxa-alkyl(meth)acrylate durch Erhitzen in die Verbindung (I) und Alkanol spaltet.

Die Stufe a) des Verfahrens, nämlich die Umsetzung von Aminoalkanolen, Amino-oxa-alkanolen und Amino-polyoxa-alkanolen mit Harnstoff und Alkanolen zu N-ω-Hydroxi-carbaminsäureestern ist neu. Diese Verbindungen wurden bisher meist durch Umsetzen von Aminoalkanolen mit Chlorkohlensäureestern gewonnen. So offenbart z.B. die US-PS 2 485 855 die Herstellung von 2-Hydroxiethylcarbamidsäureethylester aus 2-Aminoethanol und Chlorkohlensäureethylester. Nach Iwakura (Chem. High Polymers Japan 2 (1945), 305) erhält man beim Erwärmen von 6-Hydroxihexanoylazid mit Ethanol in Benzol N-(5-Hydroxipentyl)carbaminsäureethylester. Die US-PS 1 927 858 schließlich lehrt die Bildung von N-Hydroxialkylcarbaminsäureestern aus Aminoalkoholen und Dialkylcarbonaten. Alle vorgenannten Verfahren verwenden verhältnismäßig toxische und teure Edukte.

Es ist überraschend, daß die N-ω-Hydroxialkyl-carbaminsäureester nach dem erfindungsgemäßen Verfahren in guter Ausbeute erhalten werden, zumal im Hinblick auf die Bifunktionalität der Aminoalkoholkomponente eine Vielzahl verschiedener Reaktionsprodukte denkbar ist. Selbst bei Verwendung von Aminoethanol und Aminopropanol, wo durch Ringschluß die ausschließliche Bildung von 2-Oxo-1,3-oxazolidin bzw. 2-Oxo-tetrahydro-1,3-oxazin zu erwarten war, wurden die gewünschten Carbaminsäureester, nämlich der N-(2-Hydroxiethyl)- bzw. N-(3-Hydroxipropyl)-carbaminsäureester in befriedigenden Ausbeuten erhalten.

Als Aminoalkanole werden für das neue Verfahren solche vorgezogen, deren Alkylenreste 4 bis 12, insbesondere 4 bis 8 C-Atome enthalten. Beispiele für geeignete Aminoalkanole sind 2-Aminoethanol, 3-Aminopropanol, 4-Amino-butanol, 5-Aminopentanol, 6-Aminohexanol, 3-Amino-isobutanol, 2-Amino-butanol-1, 3-Methyl-5-aminopentanol und 2,2-Dimethyl-3-aminopropanol-1. Geeignete Oxa-aminoalkanole sind z.B. 3-Oxa-5-aminopentanol-1, 3-Oxa-6-aminohexanol-1, 2,2-Dimethyl-4-oxo-7-aminoheptanol und 5-Oxa-8-aminooctanol-1. Als Polyoxaaminoalkohole sei beispielhaft 3,6-Dioxa-9-aminononanol-1 genannt. Als Alkanole werden in der Stufe a) des Verfahrens vorzugsweise 1 bis 6 C-Atome enthaltende Alkanole, wie Methanol, Ethanol, Propanol, n-Butanol, Isobutanol und Hexanol eingesetzt. Von besonderem Interesse sind Propanol und Butanol. Ferner geeignet als Alkoholkomponente sind Cycloalkanole wie besonders Cyclohexanol und araliphatische Alkohole, wie Benzylalkohol und 2-Phenylethanol sowie ferner Etheralkohole wie Methoxiethanol. Bei der Umsetzung wendet man im allgemeinen ein Molverhältnis von Aminoalkanol zu Harnstoff zu Alkanol von 1 : 0,7 bis 5 : 1 bis 100, vorzugsweise von 1 : 1 bis 1,5 : 5 bis 50 an. Dies gilt auch beim Einsatz von Amino-oxa-alkanolen und Amino-polyoxa-alkanolen der genannten Art. Bei der praktischen Durchführung des Verfahrens können die Ausgangskomponenten in dem angegebenen Verhältnis vermischt und meist bei Temperaturen von 170 bis 250° C, insbesondere bei 180 bis 235° C umgesetzt werden. Dabei kann man bei Drücken von 0,1 bis 50 bar arbeiten, entsprechend dem Dampfdruck des verwendeten Alkanols, doch wird vorteilhaft ein Reaktionsdruck eingestellt derart, daß die Reaktionsmischung siedet, damit der bei der Reaktion entstehende Ammoniak möglichst vollständig abgetrennt wird. Das Abtrennen von Ammoniak während der Reaktion kann auch z.B. durch Hindurchleiten von indifferenten Gasen unterstützt werden. Anstelle von Harnstoff kann bei der Reaktion auch Biuret, Triuret usw. gegebenenfalls zusammen mit Harnstoff eingesetzt werden. Anstelle von Harnstoff und Alkanol kann man auch die entsprechenden Carbaminsäureester, gegebenenfalls unter Zusatz von Alkanol und/oder Harnstoff einsetzen.

Bei dem Verfahren kann man die Umsetzung (a) auch unter Zusatz geringer Mengen Katalysatoren durchführen. Als solche kommen besonders Chloride, Bromide, Sulfate, Phosphate, Nitrate, Borate, Alkoholate, Phenolate, Sulfonate, Oxide, Oxidhydrate, Hydroxide, Carboxylate, Chelatverbindungen, Carbonate, Thiocarbamate und Dithiocarbamate vorzugsweise des Lithiums, Kalziums, Aluminiums, Zinns, Wismuts, Antimons, Kupfers, Zinks, Titans, Vanadins, Chroms, Molybdäns, Mangans, Eisens, Nickels und Kobalts in Frage. Von besonderem Interesse sind Verbindungen von Eisen, Kobalt, Nickel, Zink, Zinn und Titan.

Derartige Katalysatoren können im allgemeinen in Mengen von 0,0001 bis 0,1, vorzugsweise von 0,0005 bis 0,05 äquivalent Metallion, bezogen auf das Aminoalkanol eingesetzt werden.

Die in der Stufe a) des neuen Verfahrens gebildeten N-ω-Hydroxialkylcarbaminsäureester können nach Abdestillieren von überschüssigem Alkanol und eventuell als Nebenprodukt gebildeten Carbaminsäureestern durch Destillation im Vakuum oder durch Kristallisation gereinigt werden. In der Stufe b) werden die N-ω-Hydroxialkylcarbaminsäureester mit Estern oder Anhydriden der Acryl- und Methacrylsäure acyliert. Für die Umesterung werden beispielsweise Acrylsäuremethylester, Acrylsäureethylester, Acrylsäure-n-butylester, Methacrylsäuremethylester, -ethylester, -propylester, -n-butylester und -t-butylester verwendet. Von besonderem Interesse ist Methacrylsäuremethylester.

Bei der praktischen Durchführung der Stufe b) des Verfahrens durch Umesterung kann der Acryl- oder Methacrylester im Unterschuß oder vorzugsweise im Überschuß eingesetzt werden. Im allgemeinen setzt man, bezogen auf den in der Stufe a) erhaltenen Carbaminsäureester die 0,2 bis 10-fach molare Menge des Acryl- oder Methacrylsäureesters ein, wobei ein Überschuß von der 1,5fachen bis zur 5fachen Menge besonders bevorzugt ist. Zusätzlich kann das Reaktionsgemisch auch noch Lösungsmittel, z.B. Benzol, Toluol, Xylol, Chlorbenzol, Dioxan, Cyclohexan, n-Heptan, n-Octan, n-Nonan, n-Decan enthalten. Bevorzugt arbeitet man in Abwesenheit von Lösungsmitteln.

Im allgemeinen verwendet man bei der Umesterungsstufe einen für Umesterungen üblichen Katalysator. Als solche kommen z.B. Alkalialkoholate, wie Natriummethylat, -ethylat, -propylat, Lithiummethylat und vorzugsweise Verbindungen von Titan, Zinn und Zirkon in Betracht. Beispielhaft genannt seien Titantetramethylat, -tetraethylat, -tetrapropylat, -tetrabutylat, Dibutylzinnoxid, Dibutylzinndilaurat, Dimethoxidibutylzinn und Zirkoniumpentan-2,4-dionat.

Die Mengen der Katalysatoren liegen häufig im Bereich von 0,0005 bis 0,5 mol, vorzugsweise von 0,001 bis 0,02 mol Katalysator je mol Carbaminsäureester. Bei der Umesterung arbeitet man im allgemeinen bei Temperaturen von 50 bis 150° C, vorzugsweise von 80 bis 120° C unter Sieden des Reaktionsgemisches, wobei das bei der Umesterung freigesetzte Alkanol gegebenenfalls zusammen mit einem Anteil des Acrylsäureesters oder Methacrylsäureesters als Azeotrop aus dem Reaktionsgemisch abdestilliert wird.

Nach Durchführung der Umesterungsreaktion können der überschüssige Acryl-oder Methacrylester und gegebenenfalls Lösungsmittel z.B. durch Destillation, vorzugsweise unter vermindertem Druck, vom Reaktionsgemisch abgetrennt werden. Soweit gewünscht, kann der erhaltene ω-Alkoxycarbamoylacrylsäure-bzw. -methacrylsäureester z.B. durch Destillation unter vermindertem Druck oder durch Kristallisation gereinigt werden. Man kann jedoch auch das Reaktionsprodukt der Umesterungsstufe ohne weitere Reinigung oder gegebenenfalls nach Abtrennung des Katalysators z.B. des Titanalkoxids durch Hydrolyse und Filtration der

Spaltung gemäß Stufe c) des Verfahren zuführen.

Bei Durchführung der Acylierung mit (Meth)acrylsäureanhydrid setzt man die Edukte bevorzugt stöchiometrisch ein. Man arbeitet im allgemeinen bei Temperaturen von 80 bis 140 °C, vorzugsweise von 90 bis 110 °C. Als Katalysatoren kommen vor allem Säuren, insbesondere konzentrierte Schwefelsäure in Betracht, die in Mengen von 0,1 bis 2 Mol.% verwendet werden. Nach Abtrennen von Katalysator und (Meth)-acrylsäure z.B. durch Neutralisieren mit wäßrigen Basen, z.B. Natriumcarbonatlösung oder verdünnter Natriumlauge, kann das Rohprodukt, gegebenenfalls nach Trocknung z.B. mit Natriumsulfat oder Magnesiumsulfat, ohne weitere Reinigung in die Spaltung gemäß Stufe c) eingesetzt werden.

Um eine vorzeitige Polymerisation der $\alpha,\beta$-monoolefinisch ungesättigten Reaktionsteilnehmer zu verhindern, setzt man dem Reaktionsgemisch vorzugsweise einen üblichen Stabilisator zu. Als solche kommen z.B. Hydrochinon, Hydrochinonmonomethylether, 2,6-Ditert.-butyl-4-methylphenol, para-Nitrosophenol (?) und/oder Phenothiazin in Betracht. Darüberhinaus hat es sich als äußerst vorteilhaft erwiesen, während der Acylierung Sauerstoff oder Luft durch das Reaktionsgemisch zu leiten.

Die erhaltenen $\omega$-Alkoxycarbamoylacrylsäure- bzw. -methacrylsäureester werden thermisch in der Stufe c) des Verfahrens in Isocyanat und Alkanol gespalten. Die thermische Spaltung von Urethanen in der Gasphase in Isocyanat und Alkanol ist zwar im Prinzip bekannt, doch ist die Gasphasen-Spaltung von Estergruppen ungesättigter Carbonsäuren enthaltenden Urethanen neu. Die dabei erhaltenen guten Ausbeuten sind im Hinblick auf die hohe Polymerisationsneigung der Acryl- und Methacryl-Gruppen und weitere in Betracht kommende Nebenreaktionen, wie Esterpyrolyse, Umesterungen und Michaeladdionen überraschend. Bei der Spaltung arbeitet man meist nach Verdampfen unter vermindertem Druck in der Gasphase bei Temperaturen zwischen 300 und 500 °C, vorzugsweise in Rohrenöfen in Gegenwart temperaturbeständiger gasdurchlässiger Reaktorfüllkörper, die z.B. die Form von Perlen, Ringen, Spänen oder Wolle haben können und die aus Kohle, Eisen, Messing, Kupfer, Zink, Aluminium, Titan, Chrom, Kobalt, Nickel und/oder Quarz bestehen können. Bevorzugt werden Füllkörper aus Eisen, Messing, Zink oder Aluminium.

Die $\omega$-Alkoxycarbamoyl-acrylsäure- bzw. -methacrylsäureester können auch in flüssiger Phase, vorzugsweise in Gegenwart eines Lösungsmittels, bei Temperaturen von 150 bis 350 °C unter Zusatz von Spaltungskatalysatoren vorgenommen werden. Hierfür geeignete Spaltungskatalysatoren sind z.B. für die Spaltung von Urethanen üblichen Metalle Titan, Eisen, Kobalt, Nickel, Zink, Zirkonium, Zinn und Blei sowie deren Salze und/oder Komplexverbindungen, z.B. Titantetrabutylat, Eisen(III)chlorid, Kobaltacetat, Nickelacetat, Zinkchlorid, Zinkacetat, Zinknaphthenat, Zirkonium-2,4-pentandionat, Zinn(IV)chlorid, Dibutylzinndilaurat, Dimethoxidibutylzinn und Bleinaphthenat. Die genannten Katalysatoren können z.B. in Mengen von 0,001 bis 2 Mol.%, vorzugsweise 0,05 bis 1 Mol.%, bezogen auf den zu spaltenden Urethanoester, eingesetzt werden.

Als vorzugsweise mitzuverwendende Lösungsmittel seien beispielhaft genannt: o-Dichlorbenzol, Phthalsäurediethylester, -dibutylester, -di(2-ethylhexyl)ester, Terephthalsäuredi(2-ethylhexyl)ester, Adipinsäuredibutylester, -di(2-ethylhexyl)ester, Tetradecan, Hexadecan, Dekalin, Tetralin und Alkylbenzole mit 4 bis 15 Kohlenstoffatomen.

Vorzugsweise werden Lösungsmittel mit Siedepunkten eingesetzt, die beträchtlich über den Siedepunkten des Isocyanats und des Alkanols liegen und mit denen auch bei Spaltung unter vermindertem Druck ausreichend hohe Temperaturen erzielt werden können. Im allgemeinen soll das Lösungsmittel einen Siedepunkt aufweisen, der 20 bis 200 °C, vorzugsweise 50 bis 200 °C über den Siedepunkten des Isocyanats bei Normaldruck liegt. Es können auch Gemische unterschiedlich siedender Lösungsmittel verwendet werden, wobei das niedriger siedende Lösungsmittel einen Siedepunkt besitzen sollte, der zwischen dem des zu spaltenden Urethans und des Isocyanats liegt. Bei einer bevorzugten Ausführungsform des Verfahrens wird in der Stufe c) eine Lösung des Spaltungskatalysators im höhersiedenden Lösungsmittel unter vermindertem Druck von 1 bis 50 mbar zum Sieden erhitzt, das Urethan, gelöst in niedriger siedendem Lösungsmittel, langsam zugegeben und gleichzeitig Isocyanat, Alkanol und ein Teil des niedriger siedenden Lösungsmittels abdestilliert. Die Trennung von Alkanol, Isocyanat und Lösungsmittel kann dann z.B. durch fraktionierte Kondensation erfolgen und das erhaltene Isocyanat kann, soweit erforderlich, durch fraktionierte Destillation weiter gereinigt werden.

Die nach dem neuen Verfahren hergestellten $\omega$-Isocyanatoalkyl-(meth)acrylate der allgemeinen Formel (I) können als Ausgangsprodukte zur Herstellung von Pharmazeutika und Insektiziden sowie als Monomere bzw. Comonomere für die Herstellung von Polymerisaten und Copolymerisaten eingesetzt werden.

## Beispiel 1

In einem 1 l-Rührautoklaven mit aufgesetzter Druckkolonne und Druckregelventil werden 35,6 g 4-Aminobutanol, 26,4 g Harnstoff, 592 g n-Butanol und 68 mg Zinn(II)chlorid 5 Stunden bei 5 bar unter Rückfluß und Abnahme von Ammoniak gekocht. Man erhielt 636 g einer gelblichen Flüssigkeit. Eine Analyse des Reaktionsgemisches (Gelpermeationschromatographie, Methode des externe Standards) zeigte, daß die Umwandlung in N-(4-Hydroxibutyl)-carbaminsäurebutylester 84 % betrug.

Nach Abdestillieren von überschüssigem Butanol und Carbaminsäurebutylester wurde der Rückstand über einen Dünnschichtverdampfer destilliert. Das Produkt geht bei 140 ° C/0,1 mbar über und erstarrt in der Vorlage kristallin. Es wurden 59.7 g (79 %) N-(4-Hydroxibutyl)carbaminsäureester (Reinheit > 99 %) erhalten. Eine Probe wurde aus Essigsäureethylester umkristallisiert und schmilzt bei 51 ° C.

Beispiel 2

378 g des nach Beispiel 1 hergestellten N-(4-Hydroxibutyl)carbaminsäurebutylesters, 800 g Methacrylsäuremethylester und 8 g Titantetrabutylat wurden unter Einleiten von Luft zum Sieden erhitzt und während 3,5 Stunden über eine Füllkörperkolonne 215 g eines Gemisches aus Methanol und Methacrylsäuremethylester abdestilliert. Überschüssiger Methacrylsäureester wurde im Vakuum abdestilliert, der Rückstand in 1,5 l Diethylether/Cyclohexan (1:2) aufgenommen, 2 g Wasser zugegeben und 30 min kräftig gerührt. Der ausgefallene Feststoff wurde abgesaugt und das Filtrat am Rotationsverdampfer eingeengt. Der Rückstand (503 g) wurde ohne weitere Reinigung in die thermische Spaltung gemäß Beispielen 3 und 4 eingesetzt.

Beispiel 3

Der Versuch wurde in einem 500 ml-Kolben mit Innenthermometer und Tropftrichter (Kolben 1) durchgeführt, der über ein kurzes Übergangsstück mit einem weiteren 500 ml-Kolben (Kolben 2) verbunden ist. Kolben 2 ist über einen Rückflußkühler an eine Vakuumölpumpe mit vorgeschalteter Kühlfalle (-78 ° C) angeschlossen. In Kolben 1 wurden 200 g Phthalsäuredi(2-ethylhexyl)ester und 1 g Dibutylzinndilaurat vorgelegt, die Apparatur auf 1 mbar evakuiert, und der Inhalt von Kolben 1 zum Sieden erhitzt (195-200 ° C). Nun tropfte man innerhalb von 45 Minuten eine Lösung von 40 g des nach Beispiel 2 erhaltenen N-(4-Methacryloyloxibutyl)carbaminsäurebutylester in 40 g Phthalsäurediethylester zu. Nach beendeter Zugabe wurden innerhalb von 10 Minuten weitere 20 g Phthalsäurediethylester zugetropft. In Kolben 2 sammelten sich 84,5 g einer farblosen Flüssigkeit, die nach gaschromatographischer Analyse neben Phthalsäureestern noch 2 % Ausgangsmaterial und 18,6 % 4-Isocyanobutylmethacrylat enthalten. Dies entspricht einer Isocyanatausbeute von 55 %.

Beispiel 4

Eine Spaltapparatur bestehend aus einem Dünnschichtverdampfer, einem Spaltreaktor (zylindrisches Quarzrohr von ca. 1 l Leervolumen, bestückt mit verzinkten Blechfüllkörpern) und einer zweistufigen Dampfkondensationsvorrichtung wurde auf 1 mbar evakuiert.

Innerhalb von 4 Stunden wurden 385 g des nach Beispiel 2 erhaltenen N-(4-Methacryeoyloxibutyl)-carbaminsäurebutylesters (mit 100 ppm Phenothiazin stabilisiert) in den auf 175 ° C erhitzten Dünnschichtverdampfer eingebracht und vollständig verdampft. Die Urethandämpfe gelangten in den Spaltreaktor, dessen durchschnittliche Temperatur 365 ° C betrug. Die austretenden Spaltgase wurden in einer nachgeschalteten zweistufigen Kondensationsvorrichtung bei 30 ° C bzw. -15 ° C fraktionierend kondensiert. Im ersten Kondensator erhielt man 276 g Kondensat, das 86 % 4-Isocyanatobutylmethacrylat und 12 % Ausgangsmaterial enthielt, im zweiten Kondensator wurden 100 g Kondensat erhalten, das neben Butanol noch 6 % Ausgangsmaterial enthielt. Die Kühlfalle vor der Vakuumölpumpe enthielt weitere 7 g Butanol.

Die Selektivität der Spaltung zum Isocyanat betrug somit 96 %. Der Inhalt von Kondensator 1 wurde bei 94 bis 100 ° C (Öltemperatur)/0,1 mbar über einen Dünnschichtverdampfer destilliert. Man erhielt 206 g 4-Isocyanatobutylmethacrylat mit einer Reinheit von 98,6 %.

Beispiel 5

In einem 1 l-Rührautoklaven mit aufgesetzter Druckkolonne und Druckregelventil wurden 42 g 5-Amino-3-oxapentanol, 26,4 g Harnstoff und 592 g n-Butanol 2 Stunden bei 230 ° C und 16 bar unter Abnahme von

Ammoniak am Rückfluß gekocht. Man erhielt 628 g einer gelblichen Flüssigkeit, deren Analyse mittels Gelpermeationschromatographie eine Umwandlung von 92 % anzeigte. Nach Abdestillieren von überschüssigem Butanol und Carbamat wurde der Rückstand von vier Ansätzen destilliert. Das Produkt geht bei 132° C/0,1 mbar über. Es wurden 286 g (87 %) N-(5-Hydroxi-3-oxapentyl)-carbaminsäurebutylester erhalten.

Beispiel 6

410 g des nach Beispiel 5 erhaltenen N-(5-Hydroxi-3-oxapentyl)-carbaminsäurebutylester, 308 g Methacrylsäureanhydrid und 0,5 g konzentrierte Schwefelsäure und 0,1 g Phenothiazin wurden unter Einleiten von Luft auf 100° C erwärmt und 6 Stunden bei dieser Temperatur gerührt. Nach Entsäuern mit wäßriger Natriumcarbonatlösung und Trocknen der organischen Phase über Natriumsulfat wurde das erhaltene rohe Urethanmethacrylat ohne weitere Reinigung in die thermische Spaltung gemäß Beispiel 7 eingesetzt.

Beispiel 7

Die Spaltung wurde in der in Beispiel 4 beschriebenen Apparatur durchgeführt.

500 g des nach Beispiel 6 erhaltenen N-(5-Methacryloyloxi-3-oxapentyl)-carbaminsäurebutylesters wurden innerhalb 2 Stunden in den auf 189° C beheizten und auf 1 mbar evakuierten Dünnschichtverdampfer gegeben. Davon wurden 297 g verdampft und 203 g liefen ab. Die durchschnittliche Spalttemperatur betrug 390° C. Im bei 35° C betriebenen Kondensator 1 wurden 230 g rohes Isocyanat gesammelt. Nach Destillation über einen Dünnschichtverdampfer (95° C/0,3 mbar) erhielt man 172 g 5-Isocyanato-3-oxapentylmethacrylat (77 %) in einer Reinheit > 97 %.

**Ansprüche**

1. Verfahren zur Herstellung von ω-Isocyanatoalkyl-[meth)-acrylaten der allgemeinen Formel I

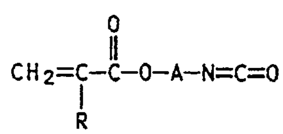

I,

in der A für einen Alkylenrest, einen Oxa-alkylenrest oder einen Polyoxaalkylenrest mit jeweils 2 bis 12 C-Atomen und R für H- oder $CH_3$-stehen, dadurch gekennzeichnet, daß man
a) ω-Aminoalkanole, ω-Amino-oxa-alkanole oder ω-Amino-polyoxa-alkanole, die jeweils 2 bis 12 C-Atome enthalten, mit Harnstoff und einem Alkanol bei 170 bis 270° C unter Abtrennung von Ammoniak zu N-ω-Hydroxyalkyl-, N-ω-Hydroxy-oxa-alkyl- oder N-ω-Hydroxy-polyoxa-alkyl-carbaminsäureestern umsetzt,
b) die N-ω-Hydroxy-carbaminsäureester durch Umsetzung mit (Meth)acrylsäurealkylestern oder (Meth)acrylsäureanhydrid verestert und
c) die erhaltenen ω-Alkoxicarbamoyl-alkyl-, -oxa-alkyl- und -polyoxa-alkyl(meth)acrylate durch Erhitzen entweder in der Gasphase, bei 300 bis 500° C, oder in flüssiger Phase, bei 150 bis 350° C in unter Zusatz von Spaltungkatalysatoren, in die Verbindung (I) und Alkanol spaltet.

2. Verfahren gemäß Anspruch 1, dadurch gekennzeichnet, daß in der Stufe a) das Molverhältnis Aminoalkohol : Harnstoff : Alkanol 1 : 1 bis 1,5 : 5 bis 50 beträgt.

3. Verfahren gemäß Anspruch 1, dadurch gekennzeichnet, daß in der Stufe a) die Reaktionstemperatur 180 bis 235° C beträgt.

4. Verfahren gemäß Anspruch 1, dadurch gekennzeichnet, daß man in Stufe a) Aminoalkanole oder ω-Amino-oxa-alkanole mit 4 bis 8 C-Atomen einsetzt.

5. Verfahren gemäß Anspruch 1, dadurch gekennzeichnet, daß in Stufe b) Methacrylsäuremethylester als Ester verwendet wird.

## Claims

1. A process for the preparation cf an ω-isocyanato-alkyl (meth)acrylate of the formula I

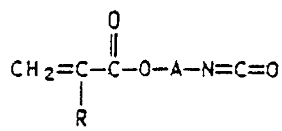

I

where A is alkylene, oxaalkylene or polyoxaalkylene, each of which is of 2 to 12 carbon atoms, and R is H or $CH_3$, wherein
   a) an ω-aminoalkanol, ω-aminooxaalkanol or ω-aminopolyoxaalkanol, each of which is of 2 to 12 carbon atoms, is reacted with urea and an alkanol at from 170 to 270−C to give an N-ω-hydroxyalkyl-, N-ω-hydroxyoxaalkyl- or Nω-hydroxypolyoxaalkyl-carbamate, ammonia being separated off,
   b) the N-ω-hydrorycarbamate is esterified by reaction with an alkyl (meth)acrylate or (meth)acrylic anhydride, and
   c) the resulting ω-alkoxycarbamyl-alkyl, -oxaalkyl or -polyoxaalkyl (meth)acrylate is cleaved to give the compound (I) and an alkanol with heating either in the gas phase at from 300 to 500−C or in the liquid phase at from 150 to 350−C, with the addition of cleavage catalysts.

2. A process as claimed in claim 1, wherein, in stage a), the molar ratio of aminoalcohol to urea to alkanol is 1 : 1 - 1,5 : 5 - 50.

3. A process as claimed in claim 1, wherein, in stage a), the reaction temperature is from 180 to 235° C.

4. A process as claimed in claim 1, wherein, in stage a), an aminoalkanol or ω-aminooxaalkanol of 4 to 8 carbon atoms is used.

5. A process as claimed in claim 1, wherein, in stage b), methyl methacrylate is used as the ester.

## Revendications

1. Procédé de préparation de <méth>acrylates d'ωisocyanatoalkyle de formule générale I

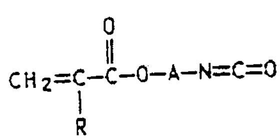

I,

dans laquelle A est mis pour un reste alkylène, un reste oxaalkylène ou un reste polyoxaalkylène avec à chaque fois de 2 à 12 atomes de carbone et R est mis pour H- ou $CH_3$-, caractérisé en ce que
   a> on fait réagir des ω-aminoalcanols, des ω-amino-oxa-alcanols ou des ω–amino–polyxaalcanols, qui contiennent chacun de 2 à 12 atomes de carbone, avec de l'urée et un alcanol à 170° -270° C avec élimination d'ammoniac pour obtenir les esters d'acide N-ω-hydroxyalkyl-. N-ω-hydroxy-oxa-alkyl- ou N- ω-hydroxypolyoxaalkyl-carbamique,
   b) on transestérifie l'ester d'acide N-ω-hydroxy-carbamique par réaction avec des <méth>acrylates d'alkyle ou l'anhydride (méth)acrylique, et
   c) on coupe le <méth>acrylate de ω-alcoxycarbamoylalkyle, -oxaalkyle et -polyoxa-alkyle, en le composé (I) et l'alcanol par chauffage soit en phase gazeuse à 300-500° C soit en phase liquide à 150-350° C avec addition de catalyseurs de coupure.

2. Procédé selon la revendication 1, caractérisé en ce que, dans l'étape a), le rapport molaire aminoalcool: urée : alcanol va de 1 : 1 à 1,5 : 5 à 50.

3. Procédé selon la revendication 1, caractérisé en ce que dans l'étape a), la température de réaction est de 180 à 235 °C.

4. Procédé selon la revendication 1, caractérisé en ce qu'on utilise dans l'étape a) des aminoalcanols ou des ω-amino-oxa-alcanols ayant de 4 à 8 atomes de carbone.

5. Procédé selon la revendication 1, caractérisé en ce que, dans l'étape b), on utilise comme ester le méthacrylate de méthyle.